# EUROPEAN PATENT APPLICATION

(11) **EP 3 696 548 A1**
(43) Date of publication of application: **19.08.2020**
(21) Application number: 19157148.8
(22) Date of filing: 14.02.2019
(51) Int. Cl.: G01N 33/68, C12Q 1/37, G01N 33/542

(54) **METHOD FOR DETERMINING A PEPTIDE FINGERPRINT OF A TARGET PROTEIN**

(71) Applicant: Nederlandse Organisatie voor toegepast- natuurwetenschappelijk onderzoek TNO, 2595 DA 's-Gravenhage (NL)
(72) Inventor: VAN DEN EERENBEEMD, Jacobus Maria Antonius, 2595 DA 's-Gravenhage (NL); CONSTANDSE, Tom Constant, 2595 DA 's-Gravenhage (NL); STORM, Arnoldus Jan, 2595 DA 's-Gravenhage (NL); LIEBIG, Thomas, 2595 DA 's-Gravenhage (NL)
(74) Representative: V.O.

(57) **Abstract**

The present disclosure concerns determining a peptide sequence of a target protein comprised in a biological sample. Accordingly, a method is provided comprising: providing the target protein; providing a molecular transporter machine; activating the molecular transporter machine within a detection volume of a detector; contacting the activated molecular transporter machine and the target protein for a time sufficient to process the target protein by the activated molecular transporter machine; and detecting with the detector a sequence of signals resulting from the processing of the target protein.

## Description

### TECHNICAL FIELD AND BACKGROUND

The present disclosure relates to methods for the determining the type of a protein as well as to the repeated use of said methods in determining the proteome of a biological sample.

Protein sequencing aims at determining the amino acid sequence of all or part of a protein. Protein sequencing may be used to identify the presence of specific, or target proteins within a biological sample. Proteins may be identified on the basis of a (partial)sequence of amino acids (e.g. a fingerprint of the protein), using a protein reference database with known sequences.

Common methods for protein sequencing are generally based on mass spectrometry methods. However, these methods are limited in that they are unsuitable to sequence complete proteins. Instead, common methods usually start out by isolating an amount of protein of interest followed by fragmentation of the protein into smaller peptides which may be analyzed using a variety of mass spectroscopy methods.

EP2872898 discloses a method capable for analyzing single proteins. In the disclosed method, proteins are functionalized with at least 2 types of fluorescent donors, selective for 2 types of predefined amino acids. Functionalized proteins are then contacted with a molecular transporter machine that is functionalized with a matching fluorescent acceptor. As the molecular transporter machine processes the protein a sequence of fluorescent signals is detected in the detection area of a microscope. This sequence forms a fingerprint signal specific to the processed protein. Specifically for ClpXP, '898 mentions the option vary the ATP concentration in throughout the system from millimolar to micromolar to slow down the process speed ClpXP to allow for adequate photon statistics and reliable analysis of time traces. The detected sequence is compared with the occurrence of such sequences in a database of proteins to determine the protein type. However, in said method, the molecular transporter machine throughout the sample volume continuously process proteins comprised within the liquid biological sample. As, the molecular transporter machine are proteases, processing of proteins results in the degradation of these proteins. This has a disadvantage that proteins may be degraded before they are analyzed. As such, the disclosed method is less suitable for analyzing proteins that are present in a relatively low abundance. The method of EP2872898 has a further disadvantage in that, molecular transporter machines within the area of detection already start processing the proteins, e.g. in a volume around their anchoring location, even when the detector, e.g. the microscope is not yet ready to start collecting signals. This may result in valuable proteins being lost for analysis, e.g. during focusing of the microscope.

The present application aims to provide a method for identifying and/or determining the type of a protein, as well as the use of molecular transporter machines in determining the type of a protein wherein one or more of the above problems are mitigated.

### SUMMARY

Aspects of the present disclosure relate to a method for determining a peptide sequence of a target protein comprised in a biological sample, particularly a liquid biological sample, e.g. proteins present in body-fluids, and/or proteins produced by a cell, or comprised within a cellular fluid or cell, in particular, in cells ranging from bacterial to human origin. Preferably, the disclosed method may be used to determine the peptide sequence of a plurality of proteins comprised in the biological sample. As such, said method may be used in determining the proteome of a biological sample. Advantageously, the method may not only be used to determine the protein sequence of proteins that are relatively abundant within the biological samples. Advantageously, the method may be especially suited to obtain a fingerprint signal specific to the processed proteins. Advantageously the obtained fingerprint signal (sequence of signals) may be obtained for proteins that are present in relatively small or minute levels compared to other proteins. Preferably, the method, e.g. the obtained fingerprint signal may be used to determine the peptide sequence of individual proteins within a biological sample, e.g. a biological sample comprising a plurality of proteins with differing structure. Accordingly, said method comprises providing a protein, e.g. a target protein comprised in a biological sample. Preferably, said protein may be provided in a liquid phase. Said method further comprises providing a molecular transporter machine, e.g. a molecular transporter machine capable of processing, e.g. transporting, the target protein. Preferably, individual molecular transporter machines process target proteins one at a time. Advantageously, processing of proteins results in the generation of a sequence of signals wherein the sequence comprises information which corresponds to a sequence of amino acids comprised in the target protein. In other words, as the molecular transporter machine processes a protein, i.e. a polypeptide, a sequence of signals relating to specific amino acids is generated as the protein is transported by or through the transporter machine.

Preferably, said molecular transporter machine is capable of processing a plurality of proteins comprised within the biological sample. In other words, preferably, the molecular transporter machine processes target proteins sequentially. By providing a molecular transporter machine capable of sequentially processing a plurality of proteins with different structures, signals may be detected for each of the processed proteins in sequence. Accordingly, the present method may be used to determine the proteome of the biological sample. Preferably, determining the proteome of a biological sample includes determining the amino acid sequence for at least part of, preferably for the whole, the target protein in the biological sample. Most preferably, determining the proteome includes determining the amino acid sequence of all proteins comprised within the biological sample.

The method for determining a peptide fingerprint, respectively determining a peptide sequence of a target protein comprised in a biological sample further comprises a step of activating the molecular transporter machine within a detection volume of a detector. Preferably, the volume in which the molecular transporter machine is activated matches the detection volume of the detector. Preferably, activation of the molecular transporter machine is essentially limited to a volume within a detection volume of a detector. In other words, preferably, the molecular transporter machine is only activated within a detection volume of a detector. By activating the molecular transporter machine only within the detection volume, processing of the target protein outside the detection volume may be reduced. Preferably, the method may be further suited to provide temporal control over the activation of the molecular transporter machine. For example, the molecular transporter machine may be activated at a specific pre-defined time, and/ or during a pre-defined time period, to match or coincide with a timing of the detector.

After activating the molecular transporter machine, the activated molecular transporter machine and the target protein are contacted for a time sufficient to process the target protein by the activated molecular transporter machine. Preferably, the contact time is selected such that the molecular transporter machine processes an entire target protein. By completely processing the target protein, the complete fingerprint signal belonging to the full amino acid sequence of that protein may be determined. More preferably, the contact time is selected such that the molecular transporter machine processes a plurality of proteins comprised within the detection volume, so that the amino acid sequence of multitude of proteins may be determined.

Advantageously, the presented method may reduce the amount, e.g. the fraction, of proteins comprised within the biological sample which are not analyzed. By minimizing the fraction of proteins that is not analyzed also proteins that are present at low relative levels may be analyzed. In other words, by minimizing the fraction of proteins that is not analyzed, the chances of not missing a specific target protein may be reduced. Advantageously, the method may also be used to determine the relative abundance of proteins comprised within the biological sample.

According to a first aspect activating involves controlling an energy supply of the molecular transporter machine. By controlling the energy supply to the molecular transporter machine, the molecular transporter machine may be switched between an inactive state when provided with insufficient energy and an active state upon provision of sufficient energy.

Alternatively or in addition, activating may involve modifying one or more of the structure of the target protein and the structure of the molecular transporter machine. For example, the structure of the target protein and/or the structure of the target protein may be modified such that initially the protein is not processed by the molecular transporter machine. This may, for example be the case when the 3D structure of the molecular transporter machine is modified such that the protein cannot be processed, e.g. cannot enter or cannot attach to the molecular transporter machine. In some embodiments processing may commence upon providing the target protein with a tag, for example in embodiments wherein the molecular transporter machine is of the ClpXP-type, processing may commence upon providing the target protein with an ssra-tag. Further, alternatively, depending on the nature of the molecular transporter machine, the proteins in the biological sample may be modified such that the protein cannot be processed, e.g. enter or attach to the transporter. Preferably, such modifications may be controllably reversed, e.g. the structure of the transporter and/or target protein may return at will from an inactive to an active structure. For example, the structure, e.g. the 3D structure, of the molecular transporter machine, may be switched from an inactive state corresponding to a first 3D structure to an active state corresponding to a second 3D structure, different from the first. Likewise, modifications to target protein may be used to modify the activity of the molecular transporter machine, e.g. from an inactive state to an active state.

### BRIEF DESCRIPTION OF DRAWINGS

These and other features, aspects, and advantages of the methods and use of various processing and activation steps of the present disclosure will become better understood from the following description, appended claims, and accompanying drawing wherein:
FIG 1 schematically depicts spatial activation of a molecular transporter machine from an inactive to an active state within a detection volume of a detector;
FIG 2 schematically depicts activation of a molecular transporter machine;
FIG 3 schematically illustrates embodiments using different local activation volumes;
FIG 4 schematically depicts processing of a target protein;
FIG 5 schematically depicts activation of a target protein for processing; and
FIG 6 schematically depicts the method for determining a peptide fingerprint of a target protein.

### DETAILED DESCRIPTION

Terminology used for describing particular embodiments is not intended to be limiting of the invention. As used herein, the singular forms "a", "an" and "the" are intended to include the plural forms as well, unless the context clearly indicates otherwise. The term "and/or" includes any and all combinations of one or more of the associated listed items. It will be understood that the terms "comprises" and/or "comprising" specify the presence of stated features but do not preclude the presence or addition of one or more other features. It will be further understood that when a particular step of a method is referred to as subsequent to another step, it can directly follow said other step or one or more intermediate steps may be carried out before carrying out the particular step, unless specified otherwise. Likewise it will be understood that when a connection between structures or components is described, this connection may be established directly or through intermediate structures unless specified otherwise.

As used herein, molecular transporter machine may be understood to be biological molecular machines. Such machines, e.g. motors, may be known as essential components for providing movement in, e.g. within, living organisms. In general terms, a motor is a device that consumes energy in one form and converts it into motion or mechanical work. As used herein, for example, molecular transporter machines (motor) may be protein-based molecular motors (machines) that move, e.g. translocate materials within a living cell. More specifically, molecular transporter machine as described herein may be defined by their function of processing (translocating, moving) polypeptides and or proteins. Proteases (called peptidase or proteinase) form a class of enzymes that typically comprise a molecular transporter machine or (sub)unit. Proteases play an important role in cells in the degradation (proteolysis) of polypeptides, e.g. proteins. Depending on the type and structure proteases may be selective towards specific proteins. During proteolysis, i.e. hydrolysis of peptide bonds, polypetides, e.g. proteins are typically translocated, e.g. moved, by a molecular motor (sub)unit towards an active catalytic center. Many different classes, e.g. using different catalytic mechanisms, of proteases exist and proteases can be found in Animalia, Plantae, Fungi, Bacteria, Archaea and viruses.

As used herein below AAA+ (or AAA) proteins turn chemical energy provided by hydrolysis of ATP into mechanical force that may exerted on a macromolecular substrate, e.g. a protein. As such AAA+ or AAA-type molecular transporter machines use energy provided by ATP to process, e.g. move(translocate) matter, e.g. proteins.

As used herein molecular transporter machines may be naturally occurring molecular transporter machines. In some embodiments it may be preferred to provide biochemically modified molecular transporter machines. For example, molecular transporter machines or sub-units thereof may be artificially linked in a configuration suitable for processing target proteins. As used herein, the phrase "ATP and or ATP analogue" refers to adenosine triphosphate (ATP) and synthetic analogue structures, e.g. compounds synthesized to study the role of ATP in biosystems so long as these structures are able to provide a molecular transporter machine with energy. Examples of ATP analogues include, but are not limited to, ATPαS, ATPβS, ATPγS, and caged ATP. Caged ATP consists of commercially available ATP units of which the phosphate structure is modified with a group that renders the ATP to be not processable by biological systems, e.g. AAA+ proteases. Removal of the functional group (uncaging), e.g. by photo excitation of a photo-cleavable group, converts the caged ATP to ATP. By uncaging the caged ATP the ATP comprised therein becomes available for processing, e.g. by ATP-ases. Examples of caged ATP include NPE-ATP, DMNPE-ATP, and Desyl-ATP.

The invention is described more fully hereinafter with reference to the accompanying drawings, in which embodiments of the invention and various activation and processing steps are shown. In the drawings, the absolute and relative sizes of systems, components, layers, and regions may be exaggerated for clarity. Embodiments may be described with reference to schematic and/or cross-section illustrations of possibly idealized embodiments and intermediate structures of the invention. In the description and drawings, like numbers refer to like elements throughout.

An aspect of the method for determining a peptide fingerprint, respectively determining a peptide sequence of a target protein comprised in a biological sample comprises spatially activating the molecular transporter machine, e.g. activating within a detection volume of a detector. Preferably, the molecular transporter machine is activated within a volume that is in communication with a detector. In other words, the molecular transporter machine is preferably activated within a detection volume of a detector. An active and/or activated molecular transporter machine 3 may be understood to include a molecular transporter machine 3 that is able to perform or is performing the specific activities belonging to said molecular transporter machine 3. In particular active molecular transporter machines 3 may process proteins or polypeptide chains. processing includes but is mot limited to binding, translocation, folding and/or unfolding and (enzymatic) reactions such as peptide-peptide bond cleaving. Inactive and/or inactive molecular transporter machines 3 may be understood to be unable to perform such processes. By activating the molecular transporter machine in the detection volume of a detector, signals, e.g. optical signals, generated by the molecular transporter machine, and/or signals relating to the processing (translocation) of the target protein may be collected by the detector. In a preferred embodiment the detection volume comprises a multitude of molecular transporter machines. By providing the detection volume with multiple molecular transporter machines, multiple signals may be detected, e.g. sequences of signals relating to the processing of multiple proteins, e.g. target proteins. Preferably, the signal, e.g. a sequence of signals, resulting from the processing of a specific target protein may be correlated to a specific molecular transporter machine, i.e. single molecule detection. By using single molecule detection, signals originating from multiple molecular transporter machine may be processed separately, e.g. without deconvolution. By using a detector capable of spatially isolating multiple signals, e.g. optical signals, a plurality of signals may be recorded simultaneously, wherein each signal or sequence of signals may be related to the processing of individual target proteins by specific molecular transporter machines present within the detection volume of the detector.

Preferably, molecular transporter machines in a volume outside the detection volume of the detector do not process target proteins. by limiting the activity of molecular transporter machines to a volume which is in connection with the detector, processing of target proteins outside said volume may be reduced, preferably avoided. By reducing the processing of target proteins outside the detection volume, signals, e.g. sequences of signals, relating thereto are not missed by the detector. Typically, processing of target protein may result in degradation (decomposition) of the target protein. For example, in embodiments wherein the molecular transporter machine is a protease the catalytic center hydrolyses the target protein (polypeptides). This proteolysis process results in the cleaving (decomposition) of the processed protein into fragments containing a few amino acids. Once a protein is decomposed it is lost for further processing and signals, e.g. sequences of signals, may no longer be obtained. Accordingly, processing of target proteins outside the detection volume is preferable avoided. This is particularly relevant for biological samples comprising a multitude of different proteins and wherein at least one of the proteins of interest (target protein) is present in a relatively low abundance, e.g. a minority species). If, in such samples, target proteins are lost outside a detection period and/or outside a detection area, the resulting collection of signal data may not correctly reflect, e.g. comprise data of, the minority species. By activating the molecular transporter machine within a detection volume the proteome of a biological sample may be determined more accurately. It will be appreciated that the herein disclosed activating methods may further allow control over the timing of such activation. By controlling the period at which the molecular transporter machines within the detection volume are activated one may reduce loss of signals that relate to the processing of target proteins during periods wherein the detector is not (yet) set up for detection, and/or after periods of detection. Suitable ways to activate, i.e. start the processing of target proteins include: controlling an energy supply of the molecular transporter machine, and modifying one or more of the structure of the target protein and the 3D structure of the molecular transporter machine from an inactive form to an active form.

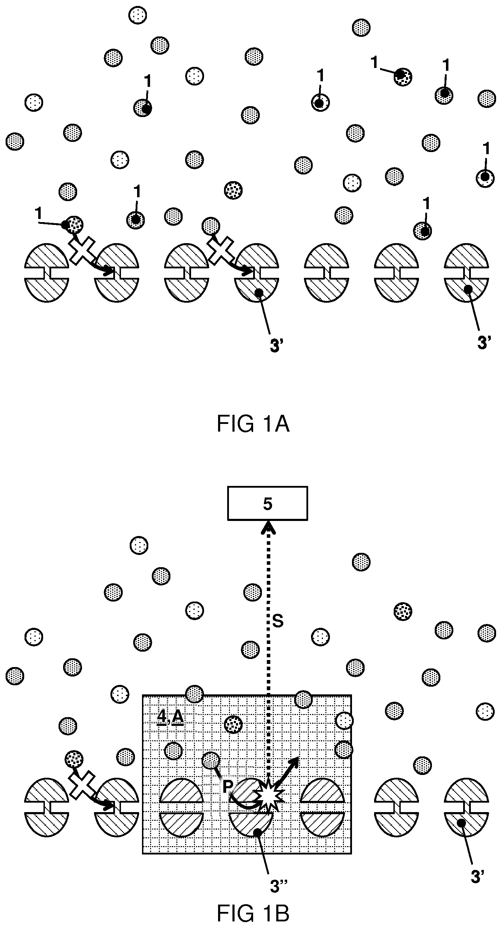

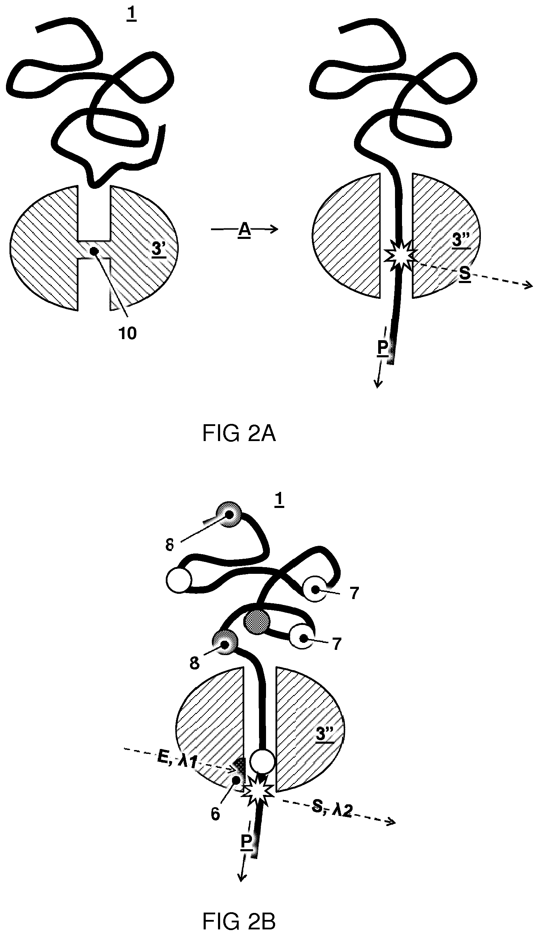

FIG 1A schematically depicts a number of different proteins 1 contacted with a plurality of inactive molecular transporter machines 3'. As illustrated, proteins 1 are not by processed by inactive molecular transporter machines 3', which is schematically depicted by crossed-out arrows. Upon activation, e.g. as shown in Fig 1B, the now activated molecular transporter machine 3" processes P (translocates) a target protein 1. This processing is schematically represented by an arrow, marked P, pointing from a target protein 1 through an open channel of the active molecular transporter machine 3. Upon processing of the target protein 1 a sequence of signals S is generated, which may be detected at a distance by a detector 5. As shown, e.g. in FIG 2A, not all molecular transporter machines are activated. Only molecular transporter machines 3 comprised within a spatially defined activation volume A are activated. Preferably, said activation volume coincides with the detection volume 4 associated with the detector (not shown).

The activation of a molecular transporter machine 3 and processing of a target protein 1 is schematically illustrated in FIG 2A. As illustrated, an inactive molecular transporter machine 3' (FIG2A, left) is unable to process (translocate) the target protein 1. As schematically shown in FIG2A this may be the result on a blocking element 10 that prevents the target protein 1 from being processed. As illustrated, blocking element 10 may be removed (switched) by an activation step A. It will be appreciated that the present disclosure envisions a number of different methods to activate a molecular transporter machine. These methods will be described in more detail below. Upon activation A, the activated molecular transporter machine 3" (FIG 2A right) may start processing target protein 1. Target protein 1, previously schematically depicted as a dot, comprises a polypetide chain. As the protein is processed P, e.g. as shown, the polypetide (protein) may be pulled (translocated) through a pore of the molecular transporter machine 3. This processing may result in the generation of a signal S, preferably a sequence of signals which relates to the structure of the processed protein. In one embodiment, formation of the signal may be the result of specific amino acids passing through the molecular transporter machine 3. In some embodiments different signals are generated for different amino acids upon translocation of the polypeptide chain. In other words, preferably processing of one more of the types of amino acids results in the generation of a signal which is specific for a type of amino acid. Recording a sequence of such signals may allow determining an order of specific amino acids comprised in the processed protein. Alternatively, or in addition the sequence, and/or parts of the sequence may be used to determine the type of protein. Preferably, the protein may be identified by comparing part(s) of a recorded signal sequence to a database comprising sequence data for a multitude of proteins. Alternatively or in addition, the processing speed may be used for determining a protein type. In some embodiments, the processing speed of the molecular transporter machine may be known and/or controlled. In other words, in embodiments wherein the number of processed amino acids per time unit is known the delay, e.g. spacing, between signals generated for a specific amino acid may provide information on the distance between these amino acids comprised in the polypeptide chain forming the target protein.

Preferably, the method for a peptide fingerprint, respectively determining a peptide sequence of a target protein makes use of naturally occurring molecular transporter machines. Particularly suitable proteins may be selected from the class known as AAA, or AAA+ proteases. By using AAA type protease the speed at which these protease process target proteins may be controllable. For example, a suitable method for controlling the speed of AAA proteins (machines) may be by controlling the level (concentration) of ATP. The activity of AAA-ases may be controlled by controlling the energy supply of these proteins. Particularly suitable proteases may be selected from the group consisting of: ClpXP, ClpAP, ClpCP, ClpEP, ClpB, ClpYq, HslUV, Lon, an archeal FtsH, PAN/20S, and the 26S proteasome.

An important subfamily of AAA+ machines function in ATP-dependent protein degradation in cells ranging from bacteria to humans. ClpXP is a relatively simple and well-characterized AAA+ protease, which serves as paradigm for other ATP-dependent proteases, including ClpAP, ClpCP, HslUV, Lon, FtsH, PAN/20S, and the 26S proteasome.

ClpXP is an archetypal AAA+ proteolytic machine and comprises two distinct protein sub units. The first is an AAA+ ATPase (called ClpX), the second a protease (called ClpP). ClpX comprises a coaxial pore formed in a hexameric ring. The ClpX unit recognizes peptide sequences (tags) of target proteins in a biological sample and proceeds to unfold the target protein and translocate the polypeptide chain through a pore and into a sequestered proteolytic compartment in ClpP. In this compartment the polypeptide is degraded into small peptide fragments. It will be appreciated that the molecular transporter machine ClpXP and its processing of target protein 1 are described herein as an example suitable for use in the disclosed method. Other proteins, e.g. the proteases listed above, performing in a similar way, e.g. translocating target proteins and generating sequences of signals may be used.

A possible mechanism of such signal generation is schematically illustrated in FIG 2B. FIG 2B schematically depicts a molecular transporter machine 3", e.g. the AAA+ ATPase sub-unit of an active ClpXP, as it processes P a target protein 1. Before processing, the specific amino acids of the target protein 1 have been selectively functionalized (labelled) with fluorophores 7,8. Likewise, the molecular transporter machine is labelled (functionalized) with a fluorophore 6. Preferably, the fluorophores 7,8 on the peptide chain and the fluorophore 6 on the molecular transporter machine form matching donor-acceptor fluorophore pairs, i.e. 6 with 7 and 6 with 8. Preferably, the first half of a donor-acceptor fluorophore pair is provided to the molecular transporter machine 3 at a location where it comes into close proximity with the matching half of the donor-acceptor fluorophore pair during processing of the labelled target protein 1. When the donor and acceptor fluorophore are in close proximity (e.g. in a near field) energy E,λ₁ of an excited donor fluorophore may be transferred to the acceptor fluorophore. This acceptor fluorophore may then emit energy (light) at a wavelength λ2, different from the excitation wavelength λ₁ of the donor fluorophore. A form of such energy transfer is known as Forster resonance energy transfer (FRET). It is believed that a donor chromophore, initially in its electronic excited state, may transfer energy to an acceptor chromophore through nonradiative dipole-dipole coupling (near filed energy transfer). Alternatively, the donor-acceptor pair may be configured to transfer an electron from an excited state of the donor to the acceptor, which may then subsequently relax upon release of a photon at a wavelength different from the excitation wavelength of the donor. A form of such electron transfer is known as Dexter electron transfer.

Accordingly, the method for determining a peptide fingerprint, respectively determining a peptide sequence of a target protein preferably comprises: labeling the target protein 1 with at least a first type of fluorophore 7 which selectively binds to a first specific predefined amino acid of the protein 1, and functionalizing, i.e. chemically modifying, the molecular transporter machine 1 with a second type of fluorophore 6, wherein the first 7 and second fluorophore 6 are suited to form a donor-acceptor fluorophore pair. Optionally or additionally the method further comprises labeling the target protein with a further type of fluorophore 8 which selectively binds to a second specific predefined amino acid of the protein 1, wherein the further fluorophore 8 is also suited to form a donor-acceptor fluorophore pair with the second fluorophore 6. In some embodiments the method further comprises exciting the donor fluorophore using light at a first wavelength. Preferably said excitation may be within the detection volume. More preferably, the excitation may be further provided specifically for a period corresponding to the time over which the molecular transporter machine 3 and target protein 1 are contacted. By limiting the excitation time the likelihood of bleaching of fluorophores may be reduced. Accordingly, the useful life-time be of molecular transporter machines may be improved. In other words, reducing the number of bleaching events may elongate the period over which signals relating to the processing of proteins may be detected. Detection of the sequence of signals preferably at least involves detection of fluorescent events over a wavelength range covering the excitation frequency (wavelength λ2) of the acceptor fluorophores 7,8, wherein the sequence of signals S is formed of a sequence of fluorescent events resulting of near-field energy transfer events between the excited donor fluorophore and the acceptor fluorophore during translocation P of the labelled target protein 1 by an activated labelled molecular transporter machine 3".

Suitable donor acceptor pairs and specific labelling methods are known in the art. Reference is made to EP2782898 and references cited therein for suitable fluorescent dyes (labels) and labelling methods. EP2782898 is hereby incorporated by reference. It will be appreciated that other fluorescent donor-acceptor pairs known in the art, e.g. in the field of FRET microscopy, may be applied as well.

Advantageously, the detection volume may preferably be within a field of view of an optical microscope. By activating the molecular transporter machine in a field of view of a microscope, signals generated during the processing of target proteins, e.g. fluorescent signals may be detected by a photodetector provided in optical connection with said field of view. Preferably, the microscope is arranged to provided with an objective with a high numerical aperture, e.g. in a range around 1.0, e.g. between 0.7 and 1.2, or between 0.8 and 1.1, for example a NA of 0.8. Providing a high NA may enable single molecule detection since with single molecule detection involves a low number of photons which need to be captured as efficiently as possible. Typically high NA objective lenses come with large magnification. A large magnification, e.g. in a range between 10x and 150x, such as 80x or 100x, may provide a spatial resolution suited to relate detected signals to individual molecular transporter machines. Providing an objective with a large NA results in a limited depth of focus (DOF).

A disadvantage of using small detection volume (e.g. by operating with one or more of a high magnification and shallow depth of view) is that only a limited number of processing events may be detected in a given period and volume. Molecular transporter machines 3 outside the detection volume continue to process target protein 1. Signals resulting from this processing are missed. Processing of target proteins may lead to damage and or (partial) degradation of the molecular transporter machine 3 and or target protein. For example, case the molecular transporter machine is a protease, the processed target protein 1 are degraded. As a result, the amino acid sequence of these processed proteins may no longer be determined. Known FRET imaging techniques, e.g. the method described in EP2872898, are subject to this effect and accordingly detected signals, e.g. protein sequences and/or identified proteins, may not accurately reflect the composition of the biological samples.

According to a first aspect of the present disclosure this problem may be mitigated by controlling the energy supply of the molecular transporter machine 3. By controlling, e.g. specially controlling, the energy supply, molecular transporter machines outside the detection volume 4 may be kept in less active, preferably inactive state, thereby reducing the number of target proteins that are processed. Processing of proteins may be reduced by depriving the molecular transporter machine 3 from one or more of the target protein and its energy supply. Accordingly controlling the energy supply preferably includes activating the energy supply, e.g. providing energy to the molecular transporter machine 3 from an initial low energy level, e.g. zero, to an energy level sufficient to activate the molecular transporter machine 3. By contacting the molecular transporter machine 3, target protein 1 and ATP and/or an ATP analogue selectively within the detection volume 4, processing of proteins outside said detection volume 4 may be avoided. Controlling the energy level may further comprise providing energy, e.g. ATP, at a constant, preferably pre-determined, level (concentration). By controlling the concentration of ATP the process speed of the molecular transporter machine 3 may be regulated to a known speed, e.g. number of processed amino acid sequence per second.

In one embodiment, controlling the energy supply may comprise: providing a sample holder, e.g. a microscopy slip, with a microfluidic channel; providing the ATP and/or an ATP to the detection volume 4 via the microfluidic channel. By providing the ATP and/or an ATP selectively to the detection volume 4, processing of proteins within the biological sample 2 will be limited to the detection volume. Alternatively or in addition, the method may comprise providing the biological sample, e.g. the target protein 1, to the detection volume via the microfluidic channel. The target protein 1 and/or ATP and/or an ATP analogue may be provided to the detection volume 4 sequentially. Optionally, a sample holder, e.g. a microcopy slip, may be provided with a plurality of microfluidic channels arranged to deliver material to the detection volume 4.

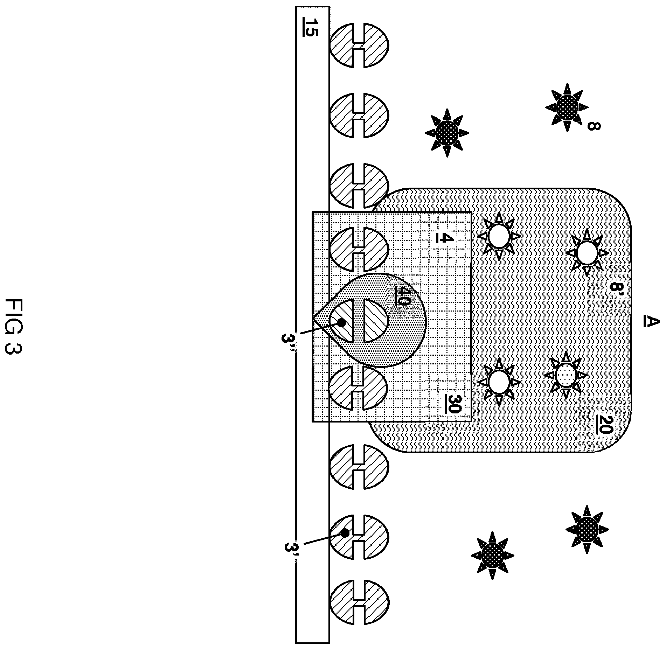

Alternatively or in addition, controlling the energy supply of the molecular transporter machine 3 may comprise locally generating ATP and/or an ATP analogue. By locally generating ATP and/or an ATP analogue, e.g. in a volume within the detection volume 4, molecular transporter machines outside that volume may be kept in less active, preferably inactive state may. In other words, by generating the energy for processing the proteins within the detection volume, molecular transporter machines outside this volume may be deprived of energy and may stop processing proteins. FIG 3 schematically illustrates embodiments of activating an energy supply of molecular transporter machines that are provided on the surface of an optically transparent substrate 15.

In a preferred embodiment, locally generating ATP and/or an ATP analogue may comprise: providing caged ATP and/or an ATP analogue 8, followed by photo activating the caged ATP and/or an ATP analogue' using light that has an energy suitable to photo activate the caged ATP and/or the ATP analogue.

Alternatively or in addition, locally generating ATP and/or an ATP analogue may comprise: providing chlorophyll (not shown), followed by, photo activating the chlorophyll to allow the chlorophyll to generate (provide) ATP to the detection volume.

Photo activation of caged ATP and/or an ATP analogue 8 and/or photo activation of chlorophyll is preferably performed within the detection volume 4. Energy provided within this activation volume 4 may be used to activate the molecular transporter machine 3. Light, at a frequency to activate the caged ATP and/or an ATP analogue 8 and/or chlorophyll may be provided to the detection volume 4 via the same objective through which signals S are collected. By providing the light via the objective that is used to collect generated signals S the activation volume and detection volume overlap. Alternatively or in addition the activation volume may be increased by scanning, e.g. moving, the objective relative to the detection volume. By increasing the activation volume additional energy (ATP) may be provided, e.g. in an area without molecular transporter machines. Additionally generated ATP and/or an ATP analogue may transport, e.g. diffuse, to molecular transporter machines within the detection volume. Alternatively or in addition, light to activate the energy supply may be provided via an alternative light path. For example, energy to activate caged ATP and/or an ATP analogue 8 may be provided via a near field interaction volume 40 of an total internal reflection light path. Alternatively light may be supplied via a separate objective and/or prism. For example, if an optically transparent sample holder 15 is used, light to activate the molecular transporter machine 3 may be supplied for an opposite direction to a separately controllable activation volume. Preferably, the activation volume overlaps with the detection volume. By selecting an objective with a smaller NA than the objective used for collecting the signals energy may be supplied over a larger volume 20. Alternatively or in addition, the light for activating the energy supply may supplied over a larger volume 20 by scanning, or stepping the focal volume of the respective objective over a desired range.

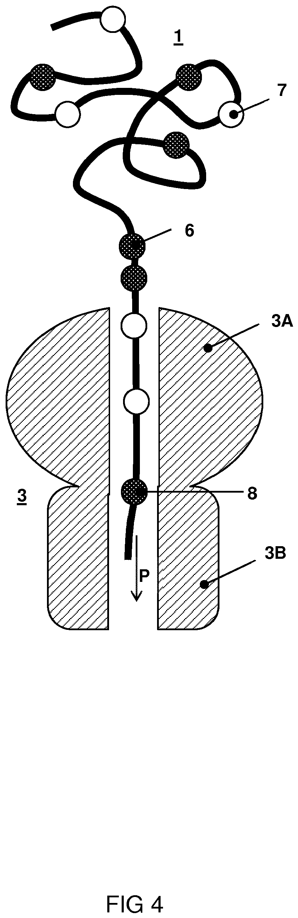

According to a second aspect of the present disclosure undesired processing of target proteins outside a detection area may be mitigated by modifying the 3D structure of the molecular transporter machine. Accordingly, the method preferably comprises providing a modified molecular transporter machine 3. Preferably, the molecular transporter machine 3 may be modified to comprise one or more switchable gatekeeper moieties, e.g. a blocking element 10 as depicted in FIG 2A, such that the transporter machine can process the target proteins only after switching (removing) the one or more gatekeeper moieties. In embodiments wherein the molecular transporter machine is a protease, e.g. a proteolytic machine that comprises two distinct proteins: an AAA+ ATPase (motor part) and a protease, the gatekeeper moiety may be provided on either one, or both, of the parts forming the molecular transporter machine 3, so long as the gatekeeper moiety prevents the molecular transporter machine from processing the target protein 1. In particular embodiments wherein the molecular transporter machine 3 is a ClpXP the gatekeeper moiety may be provided to one or more of the ClpP (protease) and ClpX (motor) subunits. Preferably, the gatekeeper moiety comprises a photo cleavable group, e.g. like photo cleavable groups used in caged ATP 8, such as 2-nitrosoacetophenone photofragments. By irradiating the photocleavable group with light of a suitable energy, the bond between the photofragments and the molecular transporter machine 3 will be broken and the gatekeeper moiety may be broken and the gatekeeper may become detached (removed). By removing the gatekeeper moiety the molecular transporter machine 3 may be activated, and processing of target proteins may start. FIG 4 schematically represents processing P of a target protein 1, labelled with fluorophores 6,7, by ClpXP 3 comprising a ClpX sub-unit 3A, a ClpP sub-unit 3B.

In particular embodiments wherein the molecular transporter machine 3 comprises a ClpX sub-unit 3A selectively providing ATP and/or an ATP analogue may further be used to control the 3D structure, e.g. the pore formation of the ClpX unit 3A as AP is known to control (regulate) the 3D assembly of the CLpX ring structure.

In another or further preferred embodiment, activating A the molecular transporter machine may comprise switching the structure of the target protein. Typically, molecular transporter-type machines 3 recognize proteins to be processed by binding to short unstructured peptide sequences, which are called degradation tags, degrons, or recognition signals. Such molecular transporter machines 3 only process proteins provided with a suitable tag. Accordingly, some preferred embodiments of the method, comprise locally attaching to proteins a tag configured to trigger the processing of the tagged target protein. FIG 4 schematically illustrates a target protein 1 and an active molecular transporter machine 3". Alternatively, the method may comprise attaching photo-activatable tag or attaching a photo-activatable group to a tagged target protein. By locally attaching a degron and/or by locally activating a photoactivatable tag, processing of target proteins may be controlled within a predefined volume.

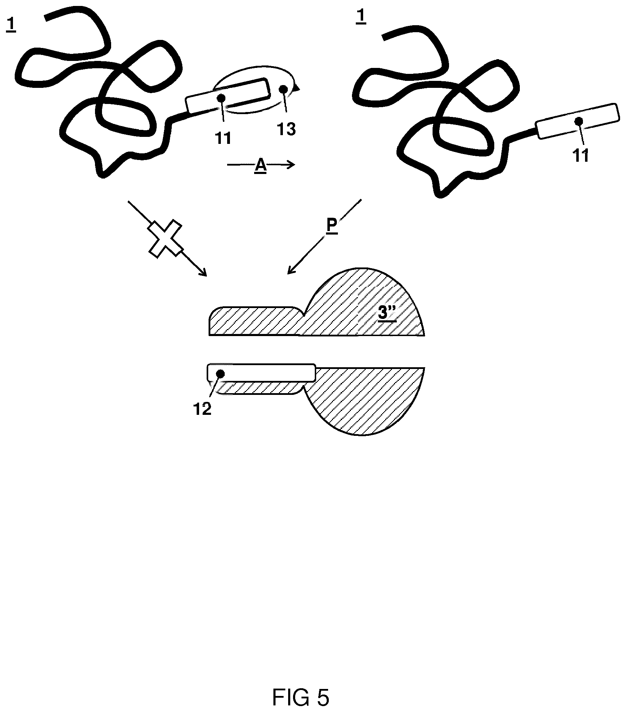

FIG 5 schematically illustrates a target protein 1 provided with a tag 11 and a molecular transporter machine 3" provided with a corresponding reception structure 12 for binding to the tag 11. In one embodiment, e.g. as shown, the tag (degron) 11 is provided with a photo cleavable group 13. With the photo cleavable group attached the protein 1 is not suited for processing by molecular transporter machine 3". Upon activation A of the target protein, e.g. photo cleavage, the blocking group 13 is removed and the target protein may be processed P by the molecular transporter machine.

In some preferred embodiments, wherein the molecular transporter machine 3 is an ClpXP the tag may be an ssra-tag. Accordingly, the ssra-tag may be provided with a photo cleavable group to allow local photo activation of the processing of tagged target proteins. It will be appreciated that molecular transporter machines, e.g. proteases comprising adaptor-mediated substrate recognition units, including local activation of such adapter units are also envisioned. More specifically, adaptor proteins, such as SspB and RssB, may work in concert with ClpXP to modulate proteolysis of specific target proteins substrates.

It will be appreciated that similar methods and techniques used to locally photo activate the energy supply may be employed to photo activate modified molecular transporter machines and/or to photo activate modified target proteins.

According to a further aspect preferred embodiments of the method may further comprise moving the detection volume from a first position to a second position. By moving the detection volume a scanning motion may be provided along the surface of a sample holder, e.g. microscopy slip. By providing a scanning motion the effective detection volume may be increased. A disadvantage of providing a scanning motion may be that molecular transporter machines 3 remain active after activating the machines within the first detection volume. Such active molecular transporter machine 3 may continue to process target proteins 1 and deplete the biological sample 2 of proteins of interest comprised. In some preferred embodiments, the method may comprise inactivating the molecular transporter machines 3 within a detection volume. In a preferred embodiment, after determining a peptide sequence at a first position and before moving to a second position, the method comprises deactivating the molecular transporter machine within the first detection volume. Deactivation may advantageously be provided by illumination. For example by using one or more of high energy and high intensity light. For some embodiments the deactivation occurs by removing the activation stimulus, e.g. releasing of the caged ATP is stopped either by exhaustion of the caged ATP or by moving the substrate which means that part is no longer illuminated.

In other or further preferred embodiments, the method may further comprise binding, e.g. via biotin-Streptavidin and/ or His-tag, the molecular transporter machine to the surface of a holder, e.g. a microscopy slip 15, e.g. as shown in FIG 3. By binding the molecular transporter machine 3 to the surface of a holder relative movement, e.g. diffusion of molecular transporter machines may be avoided. By fixing the molecular transporter machine to a substrate displacement of activated molecular transporter machines, e.g. outside a detection volume 4 may be avoided. Preferably, the molecular transporter machine 3 may be bound to a surface in a concentration such that fluorescent events (signals) from processing of target proteins may detected for individual proteins. It will be appreciated that the exact concentration. e.g. number of proteins that may be individually resolved depends of the microscope setup, e.g. the magnification that is to be used.

Optionally, the microscope may be configured to operate with super resolution techniques. The use of super resolution techniques may facilitate detection of signals S from induvial proteins at a resolution that exceeds the diffraction limit of the objective that is in use to collect the signals S. Suitable know super resolution techniques include STORM and PALM. Stochastic optical reconstruction microscopy (STORM), photo activated localization microscopy (PALM) and fluorescence photo-activation localization microscopy (FPALM) utilize sequential activation and time-resolved localization of photoswitchable fluorophores. Accordingly, for use with super resolution techniques the donor-acceptor fluorophores for use with the present methods may be selected from a group consisting of photoswitchable fluorophores. In embodiments wherein super resolution methods are used in the detection of signals S, only an optically resolvable subset of fluorophores is activated to a fluorescent state at any given moment. In this way the position of each fluorophore can be determined with a precision exceeding the diffraction limit by finding the centroid position of the single-molecule response (signal). The fluorophore is subsequently deactivated, and another subset is activated. Iteration of this process allows numerous fluorophores, preferably all, to be addressed. Preferably, the switching of the donor fluorophores is done in a high enough frequency such that the translocation events of the labelled amino acids are not missed. This means that the switching frequency is governed by the dwelling time of the labelled amino acids inside the molecular transporter machine 3.

In some embodiments the method may comprise: providing a high density of proteases with inactive donor fluorophores on the surface of a holder; activating, preferably using light, a first subset of the inactive donor fluorophores within a given surface area, collecting signals S until some or all donors fluorophores are bleached, e.g. until the donors fluorophores are longer fluorescent and; activating a second subset of the inactive donor fluorophores within that area, collecting signals S until some or all donors fluorophores are bleached. Optionally, the cycle of activating and collecting may be repeated until little or no donors can be activated anymore, e.g. until signals S can no longer be detected. Inventors find that cycling the activation and data collection may provide more protein sequence information for a given area compared to a method involving a single overall activation and measurement step at a given area.

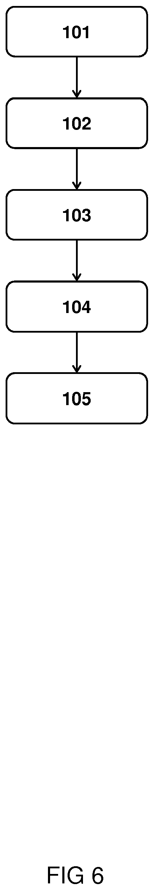

FIG 6 schematically depicts an embodiment of the method for determining a peptide fingerprint, respectively determining the peptide sequence of a target protein 1 comprised in a biological sample 2. In one embodiment, e.g. as shown, the method comprises: a step 101 of providing the target protein; a step 102 of providing the molecular transporter machine 3, a step 103 of activating A the molecular transporter machine within a detection volume 4 of the detector; a step 104 of contacting the activated molecular transporter machine and the target protein for a time sufficient to process the target protein by the activated molecular transporter machine; and a step 105 of detecting with the detector a sequence of signals S resulting from the processing P of the target protein 1.

According to a further aspect the present disclosure relates to the use of spatial activation of a molecular transporter machine in determining the proteome of a biological sample. By limiting the activity of molecular transporter machines 3 to a spatially defined volume, e.g. within a detection volume of a microscope, the proteome of a biological sample may be determined more accurately.

Additionally, the present disclosure relates to systems, e.g. optical microscope systems configured for use with any of the methods for determining the proteome of a biological sample described hereinabove. In a preferred embodiment the microscope system comprises: a photodetector for detecting fluorescent events light resulting from processing of a target protein comprised in a biological sample by a molecular transporter machine; at least first light source to excite a donor fluorophore comprised within the detection volume. In a preferred embodiment the microscope system comprises a high aperture objective. For single molecule fluorescence detection the microscope is preferably a confocal or total internal reflection fluorescence (TIRF) setup. In another or further preferred embodiment the microscope system further comprises a second light source for activating one or more of: a light switchable gate keeper moiety; activating chlorophyll; and activating photoactivatable ATP or analogue. Notionally, the microscope system may comprise a third light source for deactivating the protease.

For the purpose of clarity and a concise description, features are described herein as part of the same or separate embodiments, however, it will be appreciated that the scope of the invention may include embodiments having combinations of all or some of the features described. The various elements of the embodiments as discussed and shown offer certain advantages, such as reducing the amount of target proteins that are unavailable for sequencing. Of course, it is to be appreciated that any one of the above embodiments or processes may be combined with one or more other embodiments or processes to provide even further improvements in finding and matching designs and advantages. It is appreciated that this disclosure offers particular advantages to determining the proteome of a biological sample, e.g. identification and quantification (e.g. relative abundance) of proteins within a biological sample using detection of fluorescent signals and in general can be applied for any application benefitting from local activation of molecular transporter machines.

In interpreting the appended claims, it should be understood that the word "comprising" does not exclude the presence of other elements or acts than those listed in a given claim; the word "a" or "an" preceding an element does not exclude the presence of a plurality of such elements; any reference signs in the claims do not limit their scope; several "means" may be represented by the same or different item(s) or implemented structure or function; any of the disclosed devices or portions thereof may be combined together or separated into further portions unless specifically stated otherwise. Where one claim refers to another claim, this may indicate synergetic advantage achieved by the combination of their respective features. But the mere fact that certain measures are recited in mutually different claims does not indicate that a combination of these measures cannot also be used to advantage. The present embodiments may thus include all working combinations of the claims wherein each claim can in principle refer to any preceding claim unless clearly excluded by context.

We hereby provide a demonstration of the single molecule FRET method according to the invention to determine an amino acid sequence of a target protein using ClpXP as an exemplary molecular transporter machine. A thiol residue of a cysteine within a ClpP chamber of CplXP is labelled with maleimide-Cy3 donor dye using methods known in the field. Target proteins with an ssra-tag that is recognized by ClpX, comprised in a biological sample, are labeled with a Cy5 fluorophore using methods known in the field. The biological sample, comprising the labelled target proteins and ATP, and ClpXP are fed through the field of view of a TIRF microscopy setup via separate microfluidic channels provided within a microcopy slip. Processing reactions and corresponding single molecule FRET signal sequences are recorded to determine amino acid sequences of the target proteins. Using a database with sequence data of known proteins, target proteins within the sample are identified. The type and relative abundance of the identified proteins form the proteome of the biological sample.

In an alternative demonstration, labelled ClpXP, labelled target, a caged ATP are contacted in a sample volume formed on a microscopy slip. Within the field of view of the microscope the caged ATP is activated to start the processing reactions. Corresponding single molecule FRET signal sequences are recorded to determine amino acid sequences of the target proteins. Signals from the entire sample volume are recorded by scanning the field of view over the microscopy slip.

In a further alternative embodiment, a photo cleavable group is provided at the entry of the ClpX pore of labeled ClpXP to render the ClpXP unable to process target proteins. Then inactivated ClpXP, labelled target, and ATP are contacted in a sample volume formed on a microscopy slip. Within the field of view of the microscope the photo cleavable group on the ClpXP is removed by photoexcitation to selectively start the processing reactions. Corresponding single molecule FRET signal sequences are recorded to determine amino acid sequences of the target proteins. Signals from the entire sample volume are recorded by scanning the field of view over the microscopy slip. After recording signals within a given area activated ClpXP molecules are deactivated by a high energy light pulse.

## Claims

1. Method for determining a peptide sequence of a target protein (1) comprised in a biological sample (2), the method comprising:
∘ providing (101) the target protein,
∘ providing (102) a molecular transporter machine (3),
∘ activating (103) the molecular transporter machine within a detection volume (4) of a detector (5),
∘ contacting (104) the activated molecular transporter machine and the target protein for a time sufficient to process the target protein by the activated molecular transporter machine,
∘ detecting (105) with the detector a sequence of signals (S) resulting from the processing of the target protein, and
wherein activating involves one or more of:
- controlling an energy supply of the molecular transporter machine,
- modifying one or more of the structure of the target protein and the 3D structure of the molecular transporter machine from an inactive form (3') to an active form (3").

2. The method according to claim 1 wherein the molecular transporter machine is an AAA+ protease selected from the group consisting of: ClpXP, ClpAP, ClpCP, ClpEP, ClpB, ClpYq, HslUV, Lon, an archeal FtsH, PAN/20S, and the 26S proteasome.

3. The method according to claims 1 or 2, wherein the method further comprises:
∘ labeling the target protein with at least a first type of fluorophore (6) which selectively binds to a first specific predefined amino acid of the protein,
∘ functionalizing the molecular transporter machine with a second type of fluorophore (7), wherein the first and second fluorophore are suited to form a donor-acceptor fluorophore pair
∘ exciting (E) the donor fluorophore within the detection volume during the contact time using light at a first wavelength (λ1),

4. The method according to any one of claim 1-3 wherein, controlling the energy supply comprises selectively introducing ATP and/or an ATP analogue (ATP^{γ}S) to the detection volume via a microfluidic channel.

5. The method according to any one of claims 1-4 wherein controlling the energy supply comprises generating ATP and/or an ATP analogue (ATP^{γ}S) by one or more of:
∘ providing caged ATP and/or an ATP analogue (8), followed by
∘ photo activating the caged ATP and/or an ATP analogue within using light that has an energy suitable to release ATP and/or the ATP analogue (8'), or
∘ providing chlorophyll, followed by,
∘ photo activating the chlorophyll (9) to allow the chlorophyll to provide ATP (8') to the detection volume (4).

6. The method according to any one of claims 1-5 wherein modifying the 3D structure of the molecular transporter machine comprises:
∘ functionalizing the transporter machine with one or more light switchable gatekeeper moieties (10) such that the transporter machine can process the target proteins only after switching the one or more gatekeeper moieties,
∘ selectively providing ATP and/or an ATP analogue to the detection volume to control the 3D structure of the molecular transporter machine (ATP controls ClpX ring/pore formation),

7. The method according to claim 6, wherein the gatekeeper moiety comprises a photo-cleavable group.

8. The method according to any one of claims 1-7, wherein switching the structure of the target protein comprises one or more of:
∘ locally attaching a tag (11) configured to trigger the processing of the tagged target protein
∘ locally photo-activating target proteins functionalized with a photo-activatable tag (11').

9. The method according to any one of claims 1-8 further comprising:
∘ moving the detection volume from a first position to a second position to provide a scanning motion to increase the effective detection volume.

10. The method according to claim 9, wherein after determining a peptide sequence at a first position and before moving to a second position the molecular transporter machine within the first detection volume is inactivated by illumination using one or more of high energy and high intensity light.

11. The method according to any one of claims 1-10 further comprising binding the molecular transporter machine to the surface of a holder (15),

12. The method according to any one of claims 1-11, wherein activating involves the evanescent field of a light path via total internal reflectance.

13. The method according to any one of claims 1-11, wherein activating involves photo activation the evanescent field (40) of a light path (41) via total internal reflectance.

14. Method for determining the proteome of a biological sample, comprising: repeating the method according to any of the preceding claims sufficient times to obtain the proteome of the biological sample.
